# EUROPEAN PATENT APPLICATION

(11) **EP 3 001 950 A1**
(43) Date of publication of application: **06.04.2016**
(21) Application number: 15802863.9
(22) Date of filing: 30.01.2015
(51) Int. Cl.: A61B 5/08, A61B 5/11

(54) **RESPIRATORY MOVEMENT MEASURING DEVICE**

(30) Priority: 03.06.2014 JP 2014127698
(71) Applicant: Ideaquest Inc., Tokyo 144-0041 (JP)
(72) Inventor: AOKI, Yoshimitsu, Tokyo 144-0041 (JP); TAMURA, Kimimasa, Tokyo 144-0041 (JP); LIU, Fumio, Hasuda-shi Saitama 349-0196 (JP); SHIMIZU, Katsumi, Hasuda-shi Saitama 349-0196 (JP); TAKI, Yoshihito, Hasuda-shi Saitama 349-0196 (JP); TAKEMURA, Yasuhiro, Tokyo 144-0041 (JP)
(74) Representative: Strehl Schübel-Hopf & Partner
(86) International application number: PCT/JP2015/052675
(87) International publication number: WO 2015/186374

(57) **Abstract**

There is provided a respiratory movement measurement apparatus that can obtain a respiratory movement signal for each region by dividing and setting a region to be evaluated in a simple manner with good reproducibility. The respiratory movement measurement apparatus emits a light flux to a body surface 32 of a subject 31, captures reflected light by a camera 35, and thereby measures a respiration rate of the subject, wherein a merkmal indicating a mark position is further set on the body surface of the subject, a mark having a reflectance different from that of the body surface is placed on the merkmal, the body surface is divided into a plurality of regions on an observed image by a dividing line passing through the mark, bright spot reflection for each of the divided regions is summed, and thereby a respiratory movement signal is obtained.

## Description

### TECHNICAL FIELD

The present invention relates to a technique for three-dimensional measurement of an object.

### BACKGROUND ART

There are very few respiratory movement measurement methods or devices so far for three-dimensional measurement of an object, particularly for measurement of a respiratory movement of a subject, where the body of the subject is divided into predetermined regions and the respiratory movement of each region is measured and evaluated. If anything, examples of such commonly used devices include a polysomnography device for use in sleep-disordered breathing diagnosis. Some of the devices are used by placing a band sensor around the chest and abdomen regions of the subject to measure the respiratory movement of respective regions.

Other studies include fiber grating (FG) respiratory movement measurement where the chest and abdomen regions are automatically divided by straight lines into a plurality of regions and the respiratory movement of each region is detected (Patent Literature 4), and monitoring of the movement of each of a plurality of points on the body of the subject using motion capture (Patent Literature 2).

### CITATION LIST

### PATENT LITERATURE

PATENT LITERATURE 1: JP-A 2013-504340 (U.S. Application Publication No. 2011/066063)
PATENT LITERATURE 2: JP-A 2005-003366 (U.S. Patent No. 7545279)
PATENT LITERATURE 3: JP-A 2005-246033
PATENT LITERATURE 4: JP-A 2002-175582 (U.S. Patent No. 7431700)
PATENT LITERATURE 5: U.S. Application Publication No. 2009/0059241

### NON PATENT LITERATURE

NON PATENT LITERATURE 1: Scientific Research Grant Program (Grant-in-Aid for Scientific Research) Research Report Project Number: 22500472 (June 10, 2013)
NON PATENT LITERATURE 2: Isabella Romagnoli et al, "Optoelectronic Plethysmography has Improved our Knowledge of Respiratory Physiology and Pathophysiology", Sensors 2008, 8, pp. 7951-7972
NON PATENT LITERATURE 3: Klemen Povsic et al, "Laser 3-D measuring system and real-time visual feedback for teaching and correcting breathing", Journal of Biomedical Optics, March 2012, Vol 17(3)

### SUMMARY OF INVENTION

### TECHNICAL PROBLEM

The typical conventional measurement method using band sensors is inductive plethysmography (RIP) for detecting a change in inductance due to a change in cross-sectional area (outer peripheral cross sectional area) in the body, and other measurement methods also detect a change in outer periphery of the body due to a change in tension. In principle, those methods cannot evaluate the left and right symmetry of the body. In addition, those measurement methods cannot be used for a case of a large deformation of an upper body of the subj ect.

The method using motion capture requires a large-scale device configuration and involves great difficulty for use in clinical practice in terms of ease of application to the subject, cost, and the like. In particular, as disclosed in NON PATENT LITERATURE 2, the method requires a large number of markers to be accurately attached to the body and hence has a problem in practical use in clinical practice in terms of ease of application to the subject, cost, and the like. In addition, NON PATENT LITERATURE 3 discloses a method of measuring height distribution of an area to be measured by projecting a plurality of line light beams, but does not consider the concept of dividing the area to be measured and measuring the respiratory movement for each divided area.

In order to solve such problems, the present invention has been made and an object of the present invention is to provide a respiratory movement measurement apparatus that divides the chest and abdomen regions of a subject into a plurality of regions and acquires a respiratory movement waveform indicating the amount of movement of each region for the purpose of understanding the difference between a respiratory movement situation of the subject and a normal respiratory movement or understanding temporal variations of the respiratory movement situation of the same subject, and more specifically, a division method capable of dividing and setting a region to be evaluated in a simple manner with good reproducibility as well as a respiratory movement measurement apparatus using the same.

### SOLUTION TO PROBLEM

For ease of region setting work for the subject and for determination of divided regions on a body surface in an anatomically clear manner with good reproducibility, a plurality of mark positions (merkmal) are set on the body surface of the subject in order to form a dividing line between left and right of the body of the subject and a dividing line between a chest region and a abdomen region. A mark made of a material whose reflectance (or absorption rate) different from that of the body surface with respect to wavelength ranges of a light flux to be used for observation is provided for each of the plurality of mark positions, thereby to obtain the image of the subject including the image of the mark. A line obtained by connecting each position of the mark image on the thus obtained image to be observed is used as a border line to divide the image of the subject into a plurality of regions.

The typical specific example includes a respiratory movement measurement apparatus wherein the body surface is irradiated with a light flux having a non-uniform intensity distribution, and a temporal change in height of each portion of the body surface is detected based on light reflected from the body surface, thereby to obtain a respiratory movement signal corresponding to a respiratory movement. The respiratory movement measurement apparatus includes an imaging device that captures the body surface and outputs an image obtained by observing the body surface, wherein a plurality of merkmal indicating a mark position is set on the body surface, a mark (such as a reflector) whose reflectance (or absorption rate) is different from that of the body surface in a wavelength range of light received by the imaging device is placed on each merkmal, the body surface is divided into a plurality of regions by a dividing line passing through the image of the plurality of marks on the observed image, and the temporal change in height of each portion is summed or averaged for each divided region, thereby to obtain the respiratory movement signal of each of the divided regions.

Here, when the temporal change in height at each portion is calculated by each of the divided regions, the region to which each of the portions belongs is detected by image processing and using the region information and information about the height at each portion measured or a change in height thereof, the amount of change for each region is obtained.

Preferably, the merkmal includes at least three points: a point on a sternum, a point near an umbilical region, and a point near a middle point between left and right anterior superior iliac spines.

Preferably, the merkmal includes at least one point of a position of a rib on an episternum or sternum, an intersection between a rib and an axillary line, and an intersection between a rib and a midclavicular line.

The respiratory movement signal is obtained by averaging the amount of change corresponding to a temporal change in height of a plurality of points on the body surface for each region. Note that the change in height may be a change in height relative to a reference height or may be a change in height for a predetermined time interval.

Of the plurality of divided regions, specific adjacent regions are merged to less than the number of divided regions, and then the respiratory movement signals of the resultant regions are displayed.

The dividing line of each region is set on the observed image by connecting (and extending) each mark image in a predetermined order.

Alternatively, an individual reflectance (or absorption rate) distribution is assigned to a mark allocated to each mark position (merkmal) or the shape or size of the mark is differentiated so as to easily identify each mark image on the observed image. Based on the identification, the dividing line is automatically set by a predetermined rule.

For example, the reflector serving as the mark is prepared by arranging retroreflective elements on the surface and another light source having a wavelength range to which the imaging device substantially has sensitivity may be disposed in a direction near the direction of the imaging device when viewed from the body surface.

### ADVANTAGEOUS EFFECTS OF INVENTION

Even in the case of any deformation in the body (particularly backbone) of the subject, the above configuration allows the mark image at each mark position (merkmal) to be captured along the deformation of the backbone. These mark positions (merkmal) are placed on positions visible on the body surface of the subject that the observer can also easily palpate, and hence a desired dividing line can be obtained with good accuracy and reproducibility.

Since the dividing line can be obtained with good reproducibility, the respiratory movement signal of each region can be obtained based on this. Therefore, mutual comparison of different regions of a subject and temporal comparison of the same region of the same subject can be made with good accuracy. Even if a different examiner sets and measures the mark, the same comparable results can always be obtained.

Note the points on the rib and the sternum, the intersection between the rib and the axillary line, and the intersection between the rib and the midclavicular line, any of which is visible, can be easily palpated, and is clear even in the case of any deformation of the body. Therefore, based on these positions and the intersections, the desired dividing line can be obtained with good reproducibility.

Therefore, the present invention can easily and effectively set a region to be evaluated and compared, and hence facilitates measurement, observation, and comparison for each region. In particular, a mark is placed at a mark position (merkmal) on the body surface, which clearly indicates at which position on the measured image the region is to be divided, and hence the region can be divided with good reproducibility. Thus, in the same manner as described above, the present invention allows measurement, observation, and comparison for each region as well as temporal comparison in the same region.

The use of the respiratory movement measurement apparatus of the present invention can obtain the respiratory movement signal corresponding to each of the divided regions, and hence the measurement region of the subject is divided and the respiratory movement of each of the divided regions can be suitably measured, evaluated, and compared.

Of these divided regions, a respiratory movement in any region is clearly identified, displayed, and compared, which allows clear comparison and evaluation of the respiratory movement in a specific portion or region. Thus, it is expected to provide more useful data for diagnosis of respiratory disease and evaluation of rehabilitation effect.

### BRIEF DESCRIPTION OF DRAWINGS

[FIG 1] FIG. 1 is a schematic view illustrating an example of mark positions on a body surface of a subject.
[FIG 2] FIG 2 is a plan view illustrating an example where images of the mark positions on the body surface of the subject are connected by straight lines to determine divided regions.
[FIG 3] FIG 3 is a schematic view illustrating an entire configuration of a respiratory movement measurement apparatus according to an embodiment of the present invention.
[FIG 4] FIG 4 is a view illustrating an image outputted by a camera according to an embodiment of the present invention.
[FIG 5] FIG. 5 is a block diagram of the respiratory movement measurement apparatus according to an embodiment of the present invention.
[FIG 6] FIG 6 is a timing chart illustrating a waveform of a respiratory movement signal outputted for each divided region according to an embodiment of the present invention.
[FIG 7] FIG. 7 is a flowchart describing an operation of the respiratory movement measurement apparatus according to an embodiment of the present invention.
[FIG. 8] FIG. 8 is a plan view illustrating another example of divided regions on the body surface of a subject different from the subject of the example in FIG 2.
[FIG 9] FIG. 9 is a plan view illustrating another example where the number of mark positions and divided regions on the body surface is greater than that of the example in FIG 1.

### DESCRIPTION OF EMBODIMENTS

Hereinafter, embodiments of the present invention will be described with reference to the accompanying drawings.

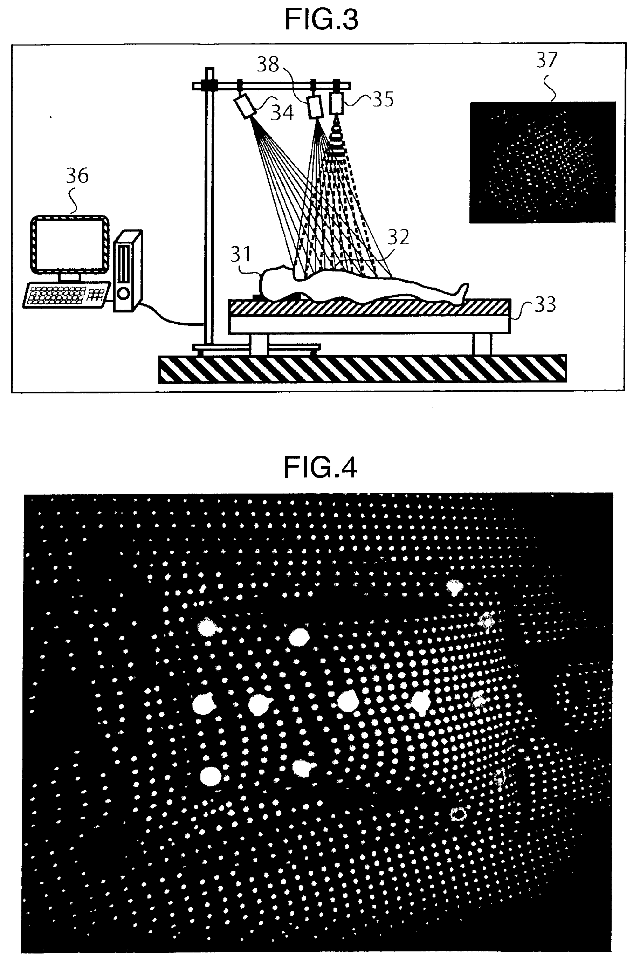

First, FIG 3 outlines the entire configuration of a respiratory movement measurement apparatus according to an embodiment of the present invention. FIG. 3 is a schematic view illustrating the entire respiratory movement measurement apparatus to which the present invention is applied. A subject 31 lies on a bed 33 with a body surface 32 facing upward. Above the subject 31, there are provided a projector 34 serving as a light source for irradiating a body surface 32 with a light flux having a non-uniform intensity distribution and a camera 35 serving as an imaging device for detecting light reflected from the body surface. The light flux, for example, has an intensity distribution having a plurality of intensity peaks in a two-dimensional array, where a plurality of bright spots is formed on the body surface 32. Output image information from the camera 35 is processed by an information processor such as a PC (personal computer) 36 disposed therenear, and the processed result is displayed on a screen of a display unit of the PC 36. Reference numeral 37 denotes an example of the screen displaying an image of a plurality of bright spots.

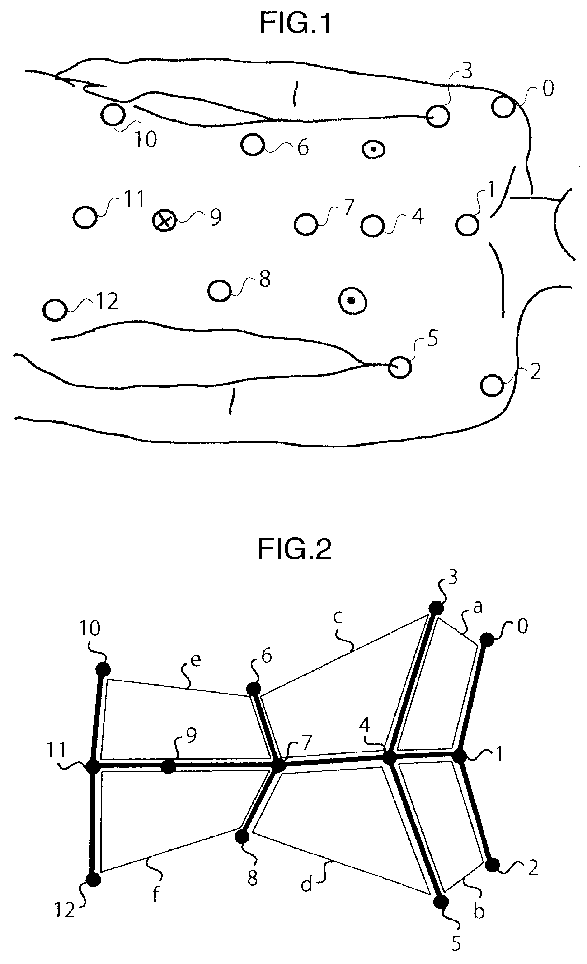

FIG. 1 is a schematic view illustrating positions of marks serving as mark positions (merkmal) such as reflectors and markers as well as divisions of regions. Examples of the mark positions (merkmal) include positions of episternum 1, left and right coracoid process 0 and 2, a point 4 on the sternum at height of the fourth rib, left and right axillaries 3 and 5, the xiphoid process 7, intersections 6 and 8 between left and right rib lower portions and the midclavicular line, the umbilical region 9, a middle point 11 between left and right anterior superior iliac spines, and left and right anterior superior iliac spines 10 and 12. Examples of the marks include things whose reflectance (or absorption rate) is different from that of the body surface in a wavelength range of light received by the imaging device. When a reflector or a marker is used as the mark, the reflector or the marker with a predetermined size is attached to a mark position (merkmal) of the body surface. Alternatively, as the mark, a coating material made of a material whose reflectance (or absorption rate) is different from that of the body surface in the wavelength range of light received by the imaging device may be applied to a mark position (merkmal) of the body surface so as to form a predetermined size.

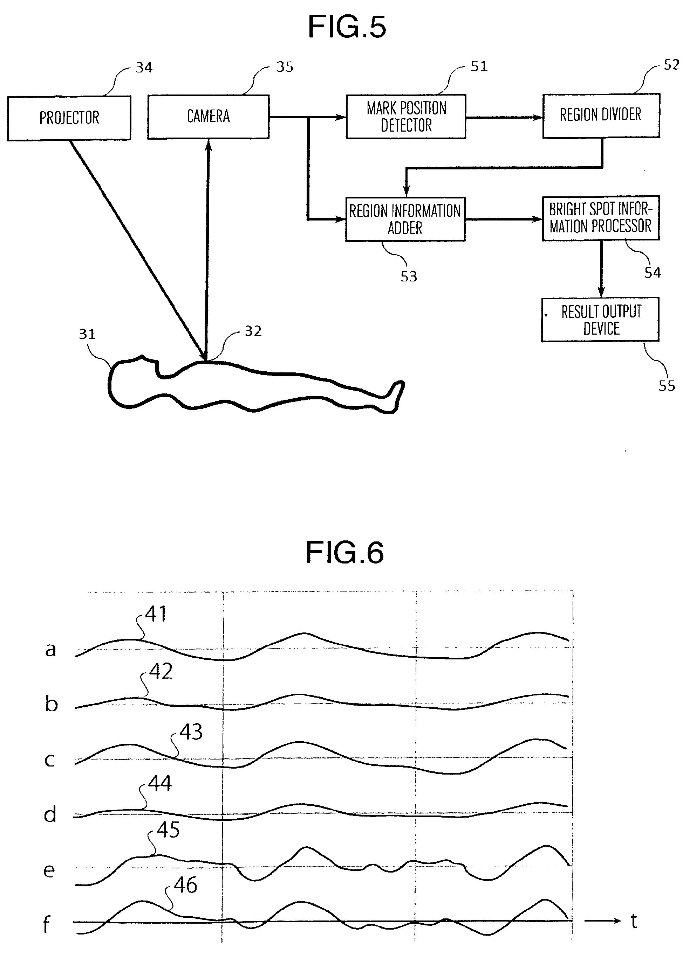

Here, FIG. 2 illustrates the region a defined by line segments connecting mark positions 0-1-4-3, the region b defined by line segments connecting mark positions 2-1-4-5, region c defined by line segments connecting mark positions 3-4-7-6, the region d defined by line segments connecting mark position 5-4-7-8, the region e defined by line segments connecting mark position 6-7-9-11-10, and the region f defined by line segments connecting mark position 8-7-9-11-12. Thus, as illustrated in FIG. 6, a respiratory movement signal for each region can be obtained.

Note that the line segments connecting from the inside (left-right center line side) of the body surface to the outside thereof such as a line segment connecting mark positions 7-6, a line segment connecting mark positions 7-8, a line segment connecting mark positions 11-10, and a line segment connecting mark positions 11-12 can be appropriately extended toward to the outside of the body surface so as to expand the respective regions on the body surface toward the side of the body surface.

FIG. 5 is a block diagram of the respiratory movement measurement apparatus adopting the present invention. The light flux emitted to the body surface 32 of the subject 31 from the projector 34 serving as the light source has a preset intensity pattern and generates a two-dimensional bright spot array on the body surface according to the present embodiment. The light reflected from the body surface is detected by the camera 35 serving as the imaging device.

At this time, illumination light for obtaining the images of the marks by the camera 35 may illuminate the entire room, but in the present embodiment, another light source 38 is disposed in a direction near the direction of the imaging device, or the camera35, when viewed from the body surface. Specifically, as illustrated in FIG 3, the light source 38 is disposed adjacent to the camera 35. Alternatively, the light source 38 may be made of a plurality of light sources and the plurality of light sources may be disposed in a ring or donut shape around the front end portion of the camera 35. The light source 38 is an incandescent lamp, a discharge lamp, a fluorescent lamp, or an LED lamp which includes a wavelength range to which the camera 35 has substantially sensitivity. When a marker whose surface has a retroreflection function is used as the mark, even the illumination light intensity is low, the reflected light intensity from the marker in the direction of the camera 35 is higher than in the other directions, and hence increases contrast to the portion other than the marker. Thus, this embodiment is effective in detecting the bright spot array as well as the markers clearly distinguished from other parts of the body surface without being inhibited by illumination of the light source 38.

A signal outputted from the camera 35 is introduced into a mark position detector 51 and a region information adder 53. Here, the camera 35 includes an imaging element having sensitivity to, for example, a wavelength range from visible light to near-infrared light, but in order to prevent unwanted light from entering as much as possible, and the camera 35 includes a bandpass filter disposed closer to the object (subject) than the imaging element so as to prevent light having wavelength ranges outside the wavelength range of the bright spot array. Thus, the wavelength range to which the camera 35 has substantially sensitivity becomes a wavelength range of light passing through the bandpass filter in the angle of view of the camera 35. In order to prevent the subject from feeling glare and reduce the effect of sunlight, the present embodiment adopts the near-infrared wavelength range.

FIG. 4 illustrates an example of signals outputted from the camera 35, or an image photograph indicating an image outputted by the camera. In this image, a large white circle indicates an image of a marker disposed at a mark position (merkmal), and a small white circle indicates an image of a bright spot.

The mark position detector 51 performs gray scaling and smoothing on the signals outputted from the camera 35 in the same manner as the bright spots, extracts the obtained smoothed image from the original image, and then binarize the image. Then, the binarized image is subjected to, for example, erosion processing three times and dilation processing three times, thereby erasing only the bright spots and leaving the images of the mark positions (merkmal).

Then, the mark position detector 51 performs contour extraction and ellipse fitting on the images at the obtained mark positions (merkmal), searches the obtained ellipse images for an ellipse image whose long axis and short axis are, for example, nine or more pixels, and outputs the center position or the position of the center of gravity of the ellipse image as the final mark position (merkmal).

A signal outputted from the mark position detector 51 is introduced into a region divider 52, where the body surface is divided into a plurality of regions in a preset order or by a method of classification based on the size, shape, or the like of the marker image on the detected mark position (merkmal) to determine the region dividing line. In addition, the signal outputted from the mark position detector 51 is sent to a region information adder 53.

The region information adder 53 classifies bright spot information received from the camera 35 for each region using information received from the region divider 52. Specifically, the region information adder 53 checks every bright spot to determine to which region the bright spot belongs and adds identification information (ID) unique to the region. More specifically, the region information adder 53 checks all bright spots to find the relationship between a bright spot and a region contour. If the bright spot is inside the region, the region information adder 53 adds the ID unique to the region.

A signal outputted from the region information adder 53 is sent to a bright spot information processor 54. The bright spot information processor 54 calculates the amount of movement of each bright spot, sums the amount of movement of all bright spots included in each region for each region, and determines the respiration rate of each region.

The result output device 55 displays the respiration rate of each region, for example, on a screen of the PC 36. FIG 6 is a graph illustrating thus obtained respiratory movement signal waveforms 41 to 46 for each of the divided regions a to f. In FIG 6, the vertical axis indicates values of each respiratory movement, namely, the respiration rate, and the horizontal axis indicates the elapsed time. Note that the horizontal lines in the waveforms 41 to 46 indicate an average reference level. Note also that S represents the most recent amplitude of the waveform 41. As used herein, the term respiration rate refers to an average displacement in a respiratory movement direction of the body surface of each region due to respiratory movement or a change in volume obtained by multiplying this by the area of the region. Note that as described above, the above-described displacement may be a displacement from a position at a predetermined time or from a reference position determined from a time average for a predetermined period of time, or may be a displacement for each predetermined time interval during movement.

In the above embodiment, the mark position detector 51, the region divider 52, the region information adder 53, the bright spot information processor 54, and the result output device 55 have been described as hardware, but these functions may be implemented as software running on the PC 36.

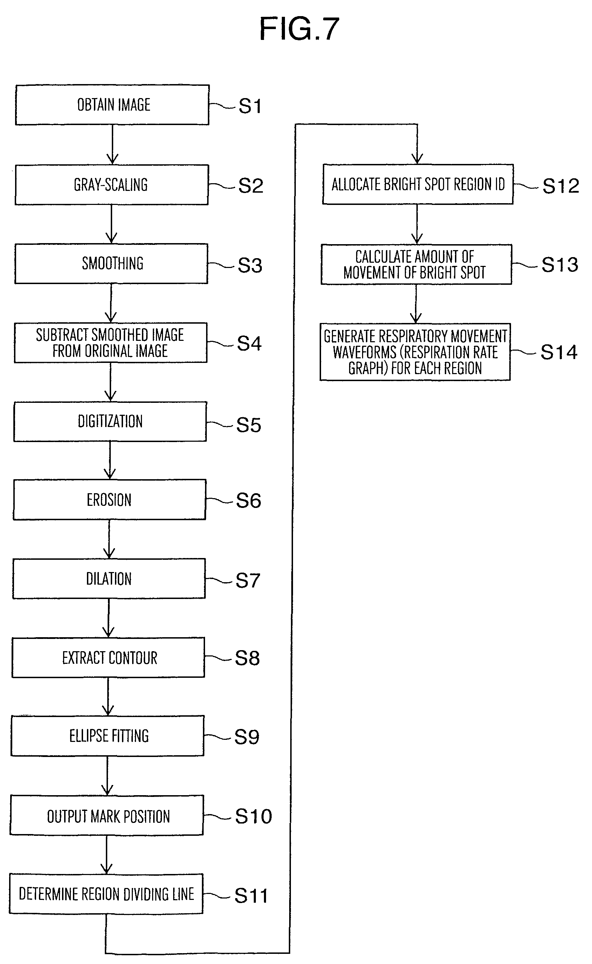

FIG 7 is a flowchart example of each function of units 51 to 55 in FIG 5 implemented by software. First, a captured image is obtained from the camera 35 (step S1), gray-scaled (step S2), and smoothed (step S2), and further the obtained smoothed image is subtracted from the original image (original image - smoothed image) (step S4), and then binarized (step S5). Further, the binarized image is subjected to, for example, erosion processing three times and dilation processing three times (steps S6 and S7), thereby extracting the contour of the image of the mark position (merkmal) (step S8). Then, ellipse fitting is performed on the extracted contour (step S9) to output the image of the mark position (merkmal) (step S10). Then, the region dividing line is determined based on the image of the mark position (merkmal) (step S11), and the corresponding region ID is allocated to each of the bright spot images obtained from the camera 35 (step S12). Then, the amount of movement of each bright spot is calculated (step S13), and then the amount of movement of all bright spots included in the region for each region is summed to determine the respiration rate for each region, thereby to generate respiratory movement waveforms (respiration rate graph) for each region (step S 14). Note that the gray-scaling (step S2) is not required if the image signal obtained in step S1 has already been gray scaled. Note also that here the ID of the region is allocated to each bright spot in advance, but another embodiment without allocating the IDs may be such that each region is searched for a bright spot contained therein, the amount of movement thereof is calculated, and the amounts are summed within the region, thereby to determine the respiration rate of each region.

Note also that in the above embodiment, the light flux from the projector 34 generates the bright spot array, but another pattern may be adopted instead of the bright spot array if the pattern is a preset intensity pattern. For example, a light flux having a random pattern or a partial pattern locally different from the other positions is obliquely emitted to the camera 35, and the reflected light is captured by the camera, thereby to detect the position of the partial pattern. In other words, if the partial pattern is different from the pattern of the other portions, the position of the partial pattern can be determined. The image of the partial pattern can be processed in the same manner as the image of the bright spot, thereby to detect the height of the position of the partial pattern or the amount corresponding to the change in height. Then, the results can be summed within the region thereby to calculate the respiration rate for each region. For example, such technique is disclosed in U.S. application publication No. 2009/0059241.

Alternatively, without providing the light flux itself from the projector 34 with a specific intensity distribution pattern like the bright spot array, each pixel of the imaging element may have a distance detection function based on time-of-flight measurement. More specifically, based on the speed of light, light propagation distance (that is, twice the distance to the object) can be detected by measuring the time from when illumination light is emitted until the imaging element receives the reflected light. In this case, the value corresponding to the height of an individual position on the body surface or to the change in height can be detected and hence the respiration rate of each region can be calculated by summing these within the region. For example, such time-of-flight measurement technique is disclosed in "Larry Li, Time-of-Flight Camera - An Introduction", Texas Instruments, Technical White Paper, SLOA1908, January 2014.

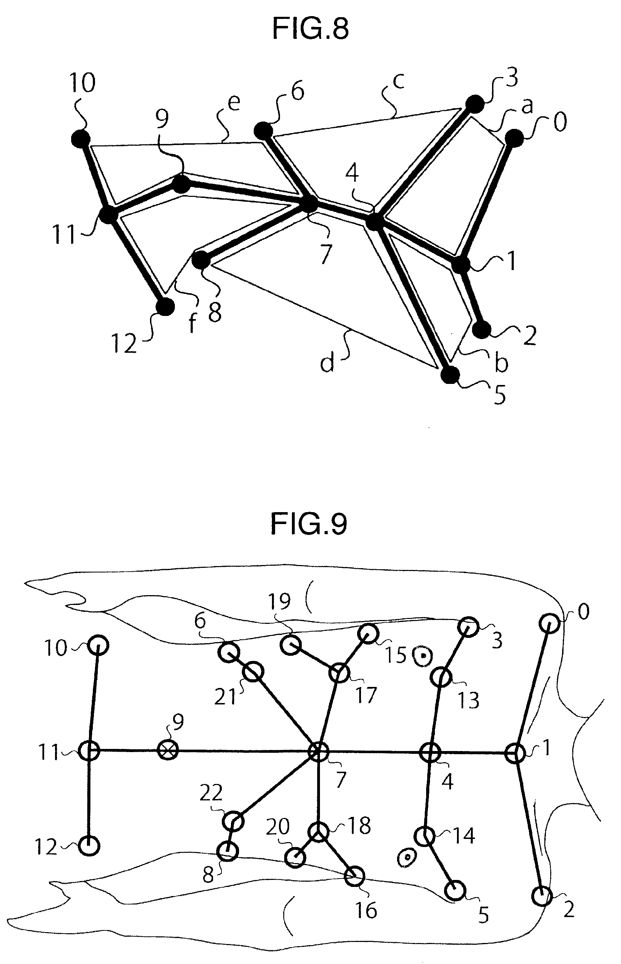

FIG 8 illustrates an example where region division using the mark positions (merkmal) is applied to a subject whose body is deformed. FIG 8 is a plan view illustrating divided regions of a subject different from the subject of the above embodiment. The body surface can be clearly divided into regions even if the body is deformed.

Table 1 lists the average values of the intraclass correlation coefficients for each region obtained by measuring the respiratory movement waveforms of a total of thirteen persons: five healthy persons, three stroke patients, three muscular dystrophy patients, and two cerebral palsy patients. More specifically, a respiratory movement waveform measurement was performed once for each of the regions 1 to 6 to measure the amplitude and obtain the intraclass correlation coefficients thereof (the results shown in ICC (1, 1); and a respiratory movement waveform measurement was performed ten times for each region to measure the amplitude and obtain the intraclass correlation coefficients of the average values (the results shown in ICC (1, 10).

**Table 1 Intraclass correlation coefficients for each region for one and ten respirations**

| | ICC (1, 1) | ICC (1, 10) |
|---|---|---|
| Region 1 | 0.64 | 0.947 |
| Region 2 | 0.65 | 0.949 |
| Region 3 | 0.666 | 0.952 |
| Region 4 | 0.496 | 0.908 |
| Region 5 | 0.823 | 0.979 |
| Region 6 | 0.846 | 0.982 |

Body deformation was recognized particularly for muscular dystrophy patients and cerebral palsy patients. The intraclass correlation coefficients ICC (1, 10) of the average values of ten times measurements for each of all regions were equal to or greater than 0.9, which indicates good reproducibility. This result indicates that the respiratory movement measurements according to the present invention can be used not only for comparison of a single subject but also for mutual comparison between a plurality of subjects.

Table 2 lists the results where the same examiner measured the same patient (subject) once on a different day, and a Bland-Altman analysis was performed on 5-minute respiratory movement amplitudes of the measurements.

**Table 2 Study of fixed bias and proportional bias in Bland-Altman analysis**

| | Region 1 | Region 2 | Region 3 | Region 4 | Region 5 | Region 6 |
|---|---|---|---|---|---|---|
| Data A | -0.564~ 0.117 | -0.323~ 0.376 | -0.445~ 0.220 | -0.415~ 0.279 | -1.557~ 0.165 | -1.713~ 0.009 |
| Data B | -0.092 | 0.085 | 0.021 | -0.101 | -0.439 | -0.352 |
| Data C | -0.307 | 0.284 | 0.069 | -0.337 | -1.62 | -1.249 |
| Data D | 2.201 | 2.201 | 2.201 | 2.201 | 2.201 | 2.201 |

| | | | | | | |
|---|---|---|---|---|---|---|
| Here, data A to data D are as follows. Data A: 95% confidence interval of average of measured value difference Data B: Correlation coefficient (r) between average and difference Data C: Value t in correlation hypothesis testing Data D: Value t in freedom degree n-2 and significant level 5% | | | | | | |

In the measured results listed in Table 2, the 95% confidence interval of average of measured value difference straddles zero in any region. In other words, some differences between the measured values are distributed evenly around 0, and the average of the measured value differences is in the vicinity of 0. Thus, it is confirmed that no significant fixed bias is observed. It is also confirmed from the correlation analysis that no significant proportional bias was observed in any region.

As described above, it is confirmed that the respiratory movement measurement apparatus of the present invention can be used to divide the body surface of various disease patients into regions with good reproducibility and measure the respiratory movement for each region.

Note that according to the respiratory movement measurement principle adopted herein, as illustrated in FIG 3, an infrared beam is divided by a two-dimensional diffraction grating into a large number of two-dimensionally arrayed light beams which are emitted from the FG projector 34 to the body surface of the subject 32. Then, a large number of bright spots occurring in a target region are captured by the video camera 35 and the subject movement is detected from the movement of each bright spot. In particular, when a fiber grating (FG) element is used as the diffraction grating for measurement, this is called an FG respiration monitor (for example, see JP-A2002-175582 (corresponding to U.S. patent No. 7431700)). Note that reference numeral 37 denotes an example of the image of the obtained bright spots.

When this measurement principle is used, an average movement in a region can be detected by summing (averaging) the movements of the bright spots contained in the region to be detected. An entire average movement can be detected by summing the movements on the body in the entire region and a movement in a region can be detected by summing the movements within the divided region.

In the above embodiment, the body surface is divided into six regions and the respiratory movement of each region is detected. This is just an example, but the body surface may be divided into fewer regions such as a chest region and an abdomen region or a right side of the body and a left side of the body, and the respiratory movement of each region may be detected. Alternatively, the body surface may be divided into finer regions to measure each region.

For example, in the present embodiment, the respiratory movements can be compared between the right side of the body and the left side of the body by generating a signal obtained by adding the amount of movements in the regions a, c, and e, and a signal obtained by adding the amount of movements in the regions b, d, and f.

Likewise, the respiratory movements can be compared between the chest side of the body and the abdomen side of the body by generating a signal obtained by adding the amount of movements in the regions a, b, c, and d, and a signal obtained by adding the amount of movements in the regions e and f. Thus, any combination of portions can be compared and the respiratory movement situation in various portions of the body can be measured.

Note that the comparison of individual regions and the comparison of regions obtained by combining several regions such as the comparison of respiratory movements between the left side and the right side, and the respiratory movements between the chest side and the abdomen side as described above may include the comparison of movement amplitudes (respiration rate) and the comparison of periodical respiratory movement phases (timings). This can clarify bad movement portions and the presence or absence of obstruction. The target movement of each region can be numerically set for pulmonary rehabilitation.

When a dividing line is drawn (specified) by connecting each merkmal on the observed image, the dividing line may be specified by using a pointing device such as a mouse to draw a line or an extended line connecting each merkmal on the observed image or using the mouse to point to the vicinity of the mutually connected merkmal.

Another embodiment may be such that the vicinity of each merkmal has been sequentially pointed to in advance by the pointing device, and the association between the connected merkmal and the pointing sequence is determined and stored, for example, in a memory in the PC 36, which allows a region border line to be automatically generated.

Alternatively, individual merkmal may be identified on an observed image by allowing each merkmal to have a specific reflectance for each mark or allowing a specific pattern to be captured. In this case, in the same manner as described above, the region border line may be automatically generated by preliminarily determining which merkmal is connected to each other. Further, instead of identifying the positional relationship of the individual merkmal by pointing to the individual merkmal or by identifying the individual merkmal on the observed image as described above, each merkmal connection may be automatically generated by using a calculation technique for use in inference and estimation such as a maximum likelihood method, a least-squares method, a genetic algorithm, and a neural network.

In the embodiment illustrated in FIG 1, the positions of the left and right coracoid process is specified by respective merkmal 0 and 2, but the positions are not limited to this embodiment. For example, a visible position between from left and right coracoid process to left and right acromion, for example, "left and right acromion" and "a middle point between left and right coracoid process and acromion" may be specified as merkmal 0 and 2 respectively.

Alternatively, the positions of left and right axillary are specified as the merkmal 3 and 5 respectively, but an intersection between any of left and right second rib to tenth rib and axillary line, for example, "an intersection between left and right third rib and axillary line" may be specified.

Further, the position on the sternum at the height of the fourth rib is specified as the merkmal 4, but a position on the sternum at the height of any of the first rib to seventh rib, for example, "a position on the sternum at the height of the third rib" may be specified.

The positions of an intersection between left and right rib lower portion and midclavicular line are specified as the merkmal 6 and 8, but for example, any positions in a range of rib lower portions such as "the lowest point of the rib lower portions" may be specified.

As illustrated in FIG. 9, the mark positions (merkmal) include the episternum 1, any points 0 and 2 from left and right coracoid process to left and right acromion, point 4 on the sternum at the height of the fourth rib, intersections 13 and 14 between left and right fourth rib and midclavicular line, intersections 3 and 5 between left and right fourth rib and axillary line, the xiphoid process 7, intersections 15 and 16 between left and right fifth rib and axillary line, intersections 17 and 18 between left and right sixth rib and midclavicular line, intersections 19 and 20 between left and right eighth rib and axillary line, intersections 21 and 22 between left and right tenth rib and midclavicular line, intersections 6 and 8 between left and right rib lower portion and axillary line, the umbilical region 9, middle point 11 between left and right anterior superior iliac spine, and positions 10 and 12 of left and right anterior superior iliac spines. The above classification can detect the movements of the portions corresponding to upper lobe, middle lobe, and lower lobe of the lung as well as the portion corresponding to the movement of the diaphragm, and hence is particularly suitable for evaluation by associating each function with the corresponding movement.

The above description has been focused on the embodiments, but the present invention is not limited to the embodiments and it is apparent to those skilled in the art that various changes and modifications may be made within the spirit of the invention and the scope of the appended claim.

### INDUSTRIAL APPLICABILITY

The present invention enables measurement and evaluation of the respiratory movement by dividing the body surface into predetermined regions although it was difficult for the prior art to measure the respiratory movement for each region. The present invention further enables mutual comparison of different regions of the subject and temporal comparison of the same region of the same subject with good accuracy. Even if a different examiner places marks for measurement, the same results can always be obtained, and hence the present invention not only can be used for conventional measurement of the respiration rate but also can promote the use in a new field such as a quantitative evaluation of rehabilitation effect that was inhibited before.

### REFERENCE SIGNS LIST

- 1: episternum
- 2, 0: any point between from left and right coracoid process to left and right acromion
- 3, 5: intersection between left and right fourth rib and axillary line
- 4: point on sternum at height of fourth rib
- 6, 8: intersection between left and right rib lower portion and axillary line
- 7: xiphoid process
- 9: umbilical region
- 10, 12: point of left and right anterior superior iliac spine
- 11: middle point of left and right anterior superior iliac spine
- 13, 14: intersection between left and right fourth rib and midclavicular line
- 15, 16: intersection between left and right fifth rib and axillary line
- 17, 18: intersection between left and right sixth rib and midclavicular line
- 19, 20: intersection between left and right eighth rib and axillary line
- 21, 22: intersection between left and right tenth rib and midclavicular line
- 31: subject
- 32: body surface
- 33: bed
- 34: projector
- 35: camera
- 36: PC
- 37: PC screen
- 51: mark position detector
- 52: region divider
- 53: region information adder
- 54: bright spot information processor
- 55: result output device

## Claims

1. A respiratory movement measurement apparatus wherein a light source emits a light flux to a body surface, a temporal change in height is detected in each of a plurality of positions on the body surface by light reflected from the body surface, and a respiratory movement signal corresponding to a respiratory movement is obtained,
the respiratory movement measurement apparatus comprising:
an imaging device that captures the body surface and outputs an observed image of the body surface;
a mark placed on each of a plurality of merkmal indicating a mark position placed on the body surface, the mark having a reflectance different from the reflectance of the body surface in a wavelength range of light received by the imaging device;
a region dividing unit that divides the body surface into a plurality of regions on the observed image outputted from the imaging device based on a dividing line passing through an image of each of the marks; and
a processing unit that obtains a respiratory movement signal of each of the divided regions based on the observed image outputted from the imaging device.

2. The respiratory movement measurement apparatus according to claim 1, wherein the light source emits a light flux having a non-uniform intensity distribution to the body surface.

3. The respiratory movement measurement apparatus according to claim 2, wherein the merkmal includes at least three points: a point on a sternum, a point near an umbilical region, and a point near a middle point between left and right anterior superior iliac spines.

4. The respiratory movement measurement apparatus according to claim 2, wherein the merkmal includes a position of a rib on an episternum or sternum, and at least one point of an intersection between a rib and an axillary line and an intersection between a rib and a midclavicular line.

5. The respiratory movement measurement apparatus according to any one of claims 2 to 4, wherein the respiratory movement signal of each of the regions is obtained by averaging an amount of change corresponding to a change in height of a plurality of points on the body surface for each of the regions.

6. The respiratory movement measurement apparatus according to any one of claims 2 to 5, wherein the respiratory movement signal is obtained for each of the regions less than the number of divided regions by merging specific regions of each of the plurality of divided regions.

7. The respiratory movement measurement apparatus according to any one of claims 2 to 6, wherein each of the marks is prepared by arranging a retroreflective element on a surface thereof, and another light source having a wavelength range to which the imaging device substantially has sensitivity is disposed in a direction near the direction of the imaging device when viewed from the body surface.

8. The respiratory movement measurement apparatus according to claim 2, wherein the processing unit detects a temporal change in height at each of a plurality of positions on the body surface using light reflected from the body surface and obtains the respiratory movement signal of each of the divided regions based on the detected results.

9. The respiratory movement measurement apparatus according to claim 8, wherein the processing unit allocates specific identification information to which divided region a pattern image of a position belongs to each of the pattern images of a plurality of positions on the body surface, calculates an amount of movement of each pattern image in each of the divided regions based on the identification information, sums the amount of movement of all pattern images contained in each region for each of the regions, and thereby determines a respiration rate for each of the regions.

10. The respiratory movement measurement apparatus according to claim 2, further comprising a display unit that displays the respiratory movement signal of each region from the processing unit.
